# EUROPEAN PATENT SPECIFICATION

(11) **EP 2 348 120 B1**
(45) Date of publication and mention of the grant of the patent: **11.06.2014**
(21) Application number: 09181016.8
(22) Date of filing: 30.12.2009
(51) Int. Cl.: C12P 7/22, C12P 41/00, C12N 9/04

(54) **Enzymatic reduction of 1-phenylpropanone and derivatives thereof**
Enzymatische Reduktion von 1-Phenylpropanon und Derivate davon
Réduction enzymatique de 1-phénylpropanone et dérivés associés

(43) Date of publication of application: 27.07.2011
(73) Proprietor: Universität Wien, 1010 Wien (AT); Technische Universität Graz, 8010 Graz (AT)
(72) Inventor: Brecker, Lothar, 1060 Wien (AT); Vogl, Michael, 1160 Wien (AT); Kratzer, Regina, 8010 Graz (AT); Nidetzky, Bernd, 8010 Graz (AT)
(74) Representative: Maiwald Patentanwalts GmbH

(56) References cited:
- WO-A1-02/057475
- CHOI YUN HEE ET AL: "Asymmetric synthesis of (S)-3-chloro-1-phenyl-1-propanol using Saccharomyces cerevisiae reductase with high enantioselectivity" APPLIED MICROBIOLOGY AND BIOTECHNOLOGY, vol. 87, no. 1, June 2010 (2010-06), pages 185-193, XP002598229
- HAMMOND ET AL: "Biocatalytic synthesis towards both antipodes of 3-hydroxy-3-phenylpropanitrile a precursor to fluoxetine, atomoxetine and nisoxetine" TETRAHEDRON LETTERS, ELSEVIER, AMSTERDAM, NL LNKD- DOI:10.1016/J.TETLET.2006.12.057, vol. 48, no. 7, 17 January 2007 (2007-01-17), pages 1217-1219, XP005834125 ISSN: 0040-4039
- DATABASE WPI Week 199240 Thomson Scientific, London, GB; AN 1992-327633 XP002598230 & JP 4 234989 A (DAICEL CHEM IND LTD) 24 August 1992 (1992-08-24)
- DATABASE WPI Week 199240 Thomson Scientific, London, GB; AN 1992-327634 XP002598231 & JP 4 234990 A (DAICEL CHEM IND LTD) 24 August 1992 (1992-08-24)
- WU X ET AL: "Enantioselective synthesis of ethyl (S)-2-hydroxy-4-phenylbutyrate by recombinant diketoreductase" TETRAHEDRON ASYMMETRY, PERGAMON PRESS LTD, OXFORD, GB, vol. 20, no. 21, 4 November 2009 (2009-11-04), pages 2504-2509, XP026791010 ISSN: 0957-4166 [retrieved on 2009-12-01]
- ANKATI, H. ET AL: "Asymetric synthesis of both antipodes of beta-hydroxy nitriles and beta-hydroxy carboxylic acids via enzymatic reduction or sequential reduction/hdrolysis" JOURNAL OF ORGANIC CHEMISTRY, vol. 74, 22 January 2009 (2009-01-22), pages 1658-1662, XP002598232
- LAVANDERA, I. ET AL.: "Stereoselective bioreduction of bulky-bulky ketones by a novel ADH from Ralstonia sp." JOURNAL OF ORGANIC CHEMISTRY, vol. 73, 3 July 2008 (2008-07-03), pages 6003-6005, XP002598233
- SALVI N A ET AL: "Asymmetric reduction of halo-substituted arylalkanones with Rhizopus arrhizus" TETRAHEDRON ASYMMETRY, PERGAMON PRESS LTD, OXFORD, GB LNKD- DOI:10.1016/J.TETASY.2008.07.035, vol. 19, no. 16, 22 August 2008 (2008-08-22), pages 1992-1997, XP025347802 ISSN: 0957-4166 [retrieved on 2008-08-29]
- LAVANDERA I ET AL: "Asymmetric anti-Prelog reduction of ketones catalysed by Paracoccus pantotrophus and Comamonas sp. cells via hydrogen transfer" TETRAHEDRON ASYMMETRY, PERGAMON PRESS LTD, OXFORD, GB LNKD- DOI:10.1016/J.TETASY.2008.08.005, vol. 19, no. 16, 22 August 2008 (2008-08-22), pages 1954-1958, XP025347795 ISSN: 0957-4166 [retrieved on 2008-08-27]
- HAGE A ET AL: "Asymmetric reduction of ketones via whole cell bioconversions and transfeR HYDRogenation: complementary approaches" TETRAHEDRON ASYMMETRY, PERGAMON PRESS LTD, OXFORD, GB LNKD- DOI:10.1016/S0957-4166(01)00172-0, vol. 12, no. 7, 8 May 2001 (2001-05-08), pages 1025-1034, XP4241685 ISSN: 0957-4166
- BROUSSY, S. ET AL.: "Enatioselective, ketoreductase-based entry into pharmaceutical building blocks: ethanol as tunable nicotinamide reductant" ORGANIC LETTERS, vol. 11, no. 2, 17 December 2008 (2008-12-17), pages 305-308, XP002598234
- KRATZER REGINA ET AL: "Whole-cell bioreduction of aromatic Î+--keto esters using Candida tenuis xylose reductase and Candida boidinii formate dehydrogenase co-expressed in Escherichia coli" MICROBIAL CELL FACTORIES, BIOMED CENTRAL, LONDON, NL LNKD- DOI:10.1186/1475-2859-7-37, vol. 7, no. 1, 10 December 2008 (2008-12-10), page 37, XP021049876 ISSN: 1475-2859
- PETSCHACHER BARBARA ET AL: "The coenzyme specificity of Candida tenuis xylose reductase (AKR2B5) explored by site-directed mutagenesis and X-ray crystallography" BIOCHEMICAL JOURNAL, vol. 385, no. Part 1, January 2005 (2005-01), pages 75-83, XP002598235 ISSN: 0264-6021
- KUMAR A ET AL: "A NEW CHEMOENZYMATIC ENANTIOSELECTIVE SYNTHESIS OF R-(-)-TOMOXETINE, (R)- AND (S)-FLUOXETINE" TETRAHEDRON LETTERS, ELSEVIER, AMSTERDAM, NL LNKD- DOI:10.1016/S0040-4039(00)85993-6, vol. 32, no. 16, 1 January 1991 (1991-01-01), pages 1901-1904, XP002269009 ISSN: 0040-4039
- JACK LIANG ET AL: 'Development of a Biocatalytic Process as an Alternative to the (-)-DIP-Cl-Mediated Asymmetric Reduction of a Key Intermediate of Montelukast' ORGANIC PROCESS RESEARCH & DEVELOPMENT vol. 14, no. 1, 15 January 2010, pages 193 - 198, XP055018991 DOI: 10.1021/op900272d ISSN: 1083-6160
- REGINA KRATZER ET AL: 'Identification of Candida tenuis xylose reductase as highly selective biocatalyst for the synthesis of aromatic ?-hydroxy esters and improvement of its efficiency by protein engineering' CHEMICAL COMMUNICATIONS no. 10, 01 January 2007, page 1047, XP055019037 DOI: 10.1039/b616475g ISSN: 1359-7345
- REGINA KRATZER ET AL: 'Probing the substrate binding site of Candida tenuis xylose reductase (AKR2B5) with site-directed mutagenesis' BIOCHEMICAL JOURNAL vol. 393, no. 1, 01 January 2006, page 51, XP055018940 DOI: 10.1042/BJ20050831 ISSN: 0264-6021

### SEQUENCE LISTING

<110> UNIVERSITÄT WIEN, TECHNISCHE UNIVERSITAT GRAZ
<120> Enzymatic reduction of 1-phenylpropanone and derivatives thereof
<130> U7174/DJB
<140> EP09181016.8
   <141> 2009-12-30
<160> 5
<170> PatentIn version 3.3
<210> 1
   <211> 322
   <212> PRT
   <213> Candida tenuis
<400> 1
   Met Ser Ala Ser Ile Pro Asp Ile Lys Leu Ser Ser Gly His Leu Met
   1               5                   10                  15
   Pro Ser Ile Gly Phe Gly Cys Trp Lys Leu Ala Asn Ala Thr Ala Gly
               20                  25                  30
   Glu Gln Val Tyr Gln Ala Ile Lys Ala Gly Tyr Arg Leu Phe Asp Gly
           35                  40                  45
   Ala Glu Asp Tyr Gly Asn Glu Lys Glu Val Gly Asp Gly Val Lys Arg
       50                  55                  60
   Ala Ile Asp Glu Gly Leu Val Lys Arg Glu Glu Ile Phe Leu Thr Ser
   65                  70                  75                  80
   Lys Leu Trp Asn Asn Tyr His Asp Pro Lys Asn Val Glu Thr Ala Leu
                   85                  90                  95
   Asn Lys Thr Leu Ala Asp Leu Lys Val Asp Tyr Val Asp Leu Phe Leu
               100                 105                 110
   Ile His Phe Pro Ile Ala Phe Lys Phe Val Pro Ile Glu Glu Lys Tyr
           115                 120                 125
   Pro Pro Gly Phe Tyr Cys Gly Asp Gly Asn Asn Phe Val Tyr Glu Asp
       130                 135                 140
   Val Pro Ile Leu Glu Thr Trp Lys Ala Leu Glu Lys Leu Val Ala Ala
   145                 150                 155                 160
   Gly Lys Ile Lys Ser Ile Gly Val Ser Asn Phe Pro Gly Ala Leu Leu
                   165                 170                 175
   Leu Asp Leu Leu Arg Gly Ala Thr Ile Lys Pro Ala Val Leu Gln Val
               180                 185                 190
   Glu His His Pro Tyr Leu Gln Gln Pro Lys Leu Ile Glu Phe Ala Gln
   195 200 205
   Lys Ala Gly Val Thr Ile Thr Ala Tyr Ser Ser Phe Gly Pro Gln Ser
   210 215 220
   Phe Val Glu Met Asn Gln Gly Arg Ala Leu Asn Thr Pro Thr Leu Phe
   225 230 235 240
   Ala His Asp Thr Ile Lys Ala Ile Ala Ala Lys Tyr Asn Lys Thr Pro
   245 250 255
   Ala Glu Val Leu Leu Arg Trp Ala Ala Gln Arg Gly Ile Ala Val Ile
   260 265 270
   Pro Lys Ser Asn Leu Pro Glu Arg Leu Val Gln Asn Arg Ser Phe Asn
   275 280 285
   Thr Phe Asp Leu Thr Lys Glu Asp Phe Glu Glu Ile Ala Lys Leu Asp
   290 295 300
   Ile Gly Leu Arg Phe Asn Asp Pro Trp Asp Trp Asp Asn Ile Pro Ile
   305 310 315 320
   Phe Val
<210> 2
   <211> 21
   <212> DNA
   <213> Artificial
<220>
   <223> Primer; D51A forward
<400> 2
   gaggcctacg gtaacgaaaa g 21
<210> 3
   <211> 21
   <212> DNA
   <213> Artificial
<220>
   <223> Primer; D51 reverse
<400> 3
   ggcaccgtcg aacaatctgt a 21
<210> 4
   <211> 22
   <212> DNA
   <213> Artificial
<220>
   <223> Primer; W24F forward
<400> 4
   ggtttcggct gtttcaaact cg 22
<210> 5
   <211> 22
   <212> DNA
   <213> Artificial
<220>
   <223> Primer; w24 reverse
<400> 5
   gatggatggc attaagtggc cg 22

## Description

### FIELD OF THE INVENTION

The present application relates to the preparation of 1-phenylpropan-1-ol and derivatives thereof, i.e. a compound of formula IIa, comprising the reduction of 1-phenylpropanone and derivatives thereof, i.e. a compound of formula I, with a xylose reductase. The present application also relates to a method further comprising the transformation of a compound of formula IIa, and to the use of a xylose reductase for the reduction of a compound of formula I.

### BACKGROUND OF THE INVENTION

Selective serotonin and norepinephrine reuptake inhibitors of the "oxetine"-group are a potent class of antidepressants which cover several representative pharmaceuticals like Fluoxetine, Norfluoxetine, Tomoxetine, Nisoxetine, Reboxetine, and Duloxetine. These compounds belong to the most important drugs used for treatment of depression, trepidation, eating disorders and psychiatric disorders. All of these compounds have been developed as racemic mixtures and most of them are still merchandised in the racemic form, even though it was shown that one of the two enantiomers usually is more potent than the other enantiomer.

There are a multitude of synthetic methods to gain racemic mixtures of *N*-methyl-3-aryloxy-3-phenyl-propylamines know in the art. For example, US 4,314,081 discloses a method for the preparation of Fluoxetine.

For preparation of enantiomerically pure *N*-methyl-3-aryloxy-3-phenyl-propylamines mostly processes using a kinetic resolution are applied. Therefore, a racemate of the *N*-methyl-3-aryloxy-3-phenyl-propylamines or of synthetic precursors is treated with an enantiomerically pure acid in order to gain a mixture of the two resulting salts, which are diastereomers to each other. These salts are separated by fractional crystallization and the desired (*R*)- or (*S*)-form of the N-methyl-3aryloxy-3-phenyl-propylamines is subsequently recovered by treatment with a strong base to liberate it from the salt. A large variety of different enantiomerically pure acids have been used for that purpose; namely mandelic acid gains good results of the resolution (WO 2008/026227; WO 2006/009884; US 7,473,804).

In other approaches lipase catalyzed hydrolysis of 3-acetoxy-3-phenylpropionitrile, ß-hydroxy-ß-arylpropionates, and of derivates thereof is used for kinetic chiral resolutions of precursors, which are useable for further synthesis of enantiomerical pure *N*-methyl-3-aryloxy-3-phenyl-propylamines.

However, in these concepts, always half of the prepared *N*-methyl-3-aryl-oxy-3-phenyl-propylamine is lost.

Synthetic chiral catalysts for transition metal catalyzed hydrogenation of carbonyl or 1,3-dicarbonyl systems are applied to directly induce chirality into prochiral precursor of *N*-methyl-3-aryloxy-3-phenyl-propylamines. In these reactions, the catalyst systems as well as the reaction conditions play an important role for the stereochemical outcome of the reaction. The enantioselectivity and the yield of transition metal catalyzed reactions are hence very sensitive to small structural changes of the reactants. Their use to synthesize a wide variety of different *N*-methyl-3-aryl-3-aryloxy-propylamines is therefore limited. In a further synthesis, enatiomerically pure and commercially available styrene oxide is utilized to gain (*R*)-and (*S*)-Fluoxetine, -Norfluoxetine and related compounds. This approach, however, only shifts the problem of gaining enantiomeric excess into a prior step of the synthesis.

Thus, there is still a need for a method of preparation of N-methyl-3-aryloxy-3-phenyl-propylamine and its derivatives in an enantiomeric excess.

### OBJECTS AND SUMMARY OF THE INVENTION

It is an objective of the present invention to provide a method for the preparation of 1-phenylpropan-1-ol and its derivatives according to formula IIa, preferably from 1-phenylpropanone, or the corresponding derivatives according to formula I, using a xylose reductase.

These and other objectives of the present invention; as they will become apparent from the ensuing description, are attained by the subject matter of the independent claims. Some of the preferred embodiments of the present invention are set forth in the dependent claims.

In a first aspect, the invention relates to a method for the preparation of a compound according to Formula IIa 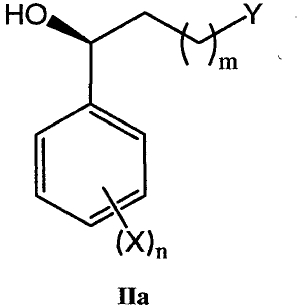 wherein
- n: is 0, 1, 2, or 3;
- X: is, independently of each other, an electron withdrawing group; and
when m = 1:
- Y: is selected from the group consisting of -H, substituted or unsubstituted -(C₁-C₆)alkyl, -F, -Cl, -Br, -I, -CN, -NH₂, amino substituted by one or two (C₁-C₆)alkyl groups, and a protected amino group; and
when m = 0:
- Y: is -CN;
comprising a step of reducing a compound according to Formula I 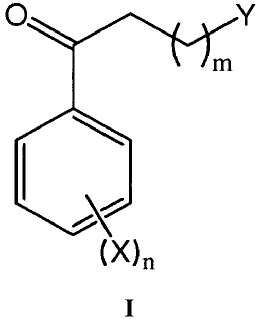 wherein n, X, m and Y are defined as above,
with a xylose reductase selected from the yeast Candida tenuis (AKR 2B5).

A second aspect of the present invention relates to a method for the preparation of a compound according to Formula III 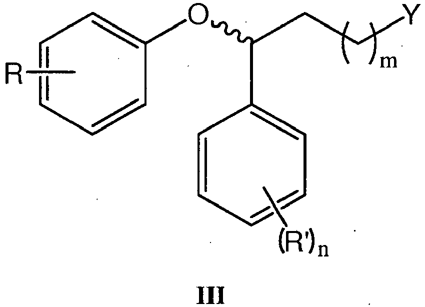 wherein
- n: is 0, 1, 2, or 3;
- R: is selected from the group consisting of substituted or unsubstituted -(C₁-C₆)alkyl, and substituted or unsubstituted -(C₁-C₆)alkoxy;
- R': is selected from the group consisting of X; and O-SO₃H, -OH, -O-C(O)CH₃, -NH₂, amino substituted by one or two -(C₁-C₆)alkyl groups, -NH-C(O)CH₃, -(C₁-C₆)alkyl, -CH₂OH, -COOH, -COO-(C₁-C₄)alkyl, -CHO, -C(O)-(C₁-C₄)alkyl; and
when m = 1:
- Y: is selected from the group consisting of -H, substituted or unsubstituted -(C₁-C₆)alkyl, -F, -Cl, -Br, -I, -CN, -NH₂, amino substituted by one or two -(C₁-C₆)alkyl groups, and a protected amino group; and
when m = 0:
- Y: is -CN;
comprising the method according to the first aspect of the invention for the preparation of a compound according to Formula IIa, and the subsequent transformation of the compound according to Formula IIa to a compound according to Formula III.

Finally, in a third aspect, the invention relates to the use of a xylose reductase selected from the yeast Candida tenuis for the reduction of a compound according to Formula I.

### FIGURE LEGENDS

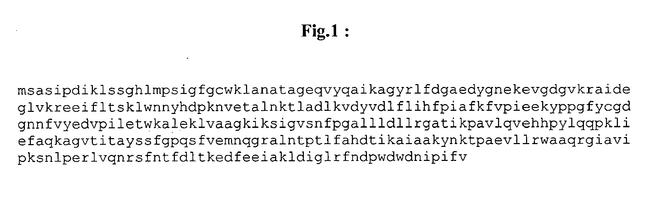

- Figure 1:: depicts the amino acid sequence of xylose reductase from *C*. *tenuis* (SEQ ID No.: 1).

### DETAILED DESCRIPTION OF THE INVENTION

Before describing in detail exemplary embodiments of the present invention, definitions important for understanding the present invention are given. It is to be understood that unless defined otherwise, all technical and scientific terms used herein have the same meanings as commonly understood by one of ordinary skill in the art.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %.

It is to be understood that the term "comprising" is not limiting. For the purposes of the present invention the term "consisting of" is considered to be a preferred embodiment of the term "comprising of". If hereinafter a group is defined to comprise at least a certain number of embodiments, this is meant to also encompass a group which preferably consists of these embodiments only.

"-(C₁-C₆)alkyl" means a straight chain or branched non-cyclic hydrocarbon having from 1 to 6 carbon atoms. Representative straight chain -(C₁-C₆)alkyls include -methyl, -ethyl, -n-propyl, -n-butyl, -n-pentyl, and -n-hexyl. Representative branched -(C1-C6)alkyls include -iso-propyl, -sec-butyl, -iso-butyl, -tert-butyl, -iso-pentyl, -neopentyl, 1-methylbutyl, 2-methylbutyl, 3-methylbutyl, 1,1-dimethylpropyl, 1,2-dimethylpropyl, 1-methylpentyl, 2-methylpentyl, 3-methylpentyl, 4-methylpentyl, 1-ethylbutyl, 2-ethylbutyl, 3-ethylbutyl, 1,1-dimethtylbutyl, 1,2-dimethylbutyl, 1,3-dimethylbutyl, 2,2-dimethylbutyl, 2,3-dimethylbutyl, and 3,3-dimethylbutyl.

"-(C₁-C₄)alkyl" means a straight chain or branched non-cyclic hydrocarbon having from 1 to 4 carbon atoms. Representative straight chain -(C₁-C₄)alkyls include -methyl, -ethyl, -n-propyl, and -n-butyl. Representative branched -(C₁-C₄)alkyls include -iso-propyl, -sec-butyl, -iso-butyl, and -tert-butyl.

"-(C₁-C₂)alkyl" means a straight chain non-cyclic hydrocarbon having 1 or 2 carbon atoms. Representative straight chain -(C₁-C₂)alkyls include -methyl and -ethyl.

"-(C₁-C₆)alkoxy" means a straight chain or branched non-cyclic hydrocarbon having one or more ether groups and from 1 to 6 carbon atoms. Especially, a -(C₁-C₆)alkoxy group may be represented by a group of-O-(C₁-C₆)alkyl. Representative straight chain and branched -(C₁-C₆)alkoxys include methoxy, ethoxy, methoxymethyl, 2-methoxyethyl, 5-methoxypentyl, 3-ethoxybutyl and the like.

"-(C₁-C₄)alkoxy" means a straight chain or branched non-cyclic hydrocarbon having one or more ether groups and from 1 to 4 carbon atoms. Especially, a -(C₁-C₄)alkoxy group may be represented by a group of -O-(C₁-C₄)alkyl. Representative straight chain and branched -(C₁-C₄)alkoxys include methoxy, ethoxy, methoxymethyl, 2-methoxyethyl, and the like.

"-(C₁-C₂)alkoxy" means a straight chain or branched non-cyclic hydrocarbon having one or more ether groups and 1 or 2 carbon atoms. Especially, a -(C₁-C₂)alkoxy group may be represented by a group of -O-(C₁-C₂)alkyl. Representative straight chain and branched -(C₁-C₂)alkoxys include methoxy, ethoxy, methoxymethyl. Any alkyl or alkoxy may be substituted by one or more second groups. A preferred substitution is the substitution with a halogen atom, an amino group, a hydroxy group, or a hydroxy-phenyl group, preferably 4-hydroxy-phenyl. An especially preferred substitution is the substitution with at least one fluoro atom, preferably several fluoro atoms.

"Halogen" or "halo" means -F (fluoro), -C1 (chloro), -Br (bromo), or -I (iodo). When a first group is "substituted" the group may be substituted with one or more second groups. When a first group is "substituted with one or more" second groups, one or more hydrogen atoms of the first group is replaced with a corresponding number of second groups. Where the number of second groups is two or greater, each second group can be the same or different.

An "amino substituted by one or two -(C₁-C₆)alkyl groups" may be expressed as a -NR¹R² group, wherein R¹ and R² are, independently of each other, selected from hydrogen and -(C₁-C₆)alkyl, with the proviso that at least one of R¹ and R² is other than hydrogen. It is preferred that the -(C₁-C₆)alkyl group is selected from a -(C₁-C₄)alkyl group, and a -(C₁-C₂)alkyl group, with the definition of all alkyl groups as above.

The term "protected amino group" shall refer to an amino group which is protected by an amine protecting group. Such a group protects the amine during reactions of the synthesis from undesired transformation and can be easily removed.

The term "amine protecting group" means an easily removable group which is known in the art to protect an amino group against undesirable reaction during synthetic procedures and to be selectively removable. The use of amine protecting groups is well known in the art for protecting groups against undesirable reactions during a synthetic procedure and many such protecting groups are known, for example, T. H. Greene and P. G. M. Wuts, Protective Groups in Organic Synthesis, 4th edition, John Wiley & Sons, New York (2007), incorporated herein by reference. Preferred amine protecting groups are acyl, including formyl, acetyl, chloroacetyl, trichloroacetyl, o-nitrophenylacetyl, o-nitrophenoxyacetyl, trifluoroacetyl, acetoacetyl, 4-chlorobutyryl, isobutyryl, o-nitrocinnamoyl, picolinoyl, acylisothiocyanate, aminocaproyl, benzoyl and the like, and acyloxy including methoxycarbonyl, 9-fluorenylmethoxycarbonyl (Fmoc), 2,2,2-trifluoroethoxy-carbonyl, 2-trimethylsilylethxoycarbonyl, vinyloxycarbonyl, allyloxycarbonyl (Alloc), t-butyloxycarbonyl (Boc), 1,1-dimethylpropynyloxycarbonyl, benzyloxycarbonyl (Cbz), p-nitrobenzyloxycarbonyl, 2,4-dichlorobenzyloxycarbonyl, and the like.

The term "electron withdrawing", as employed herein, refers to any substituent which attracts the electrons from a conjugated electron structure, thereby providing a polarized resonating structure. Illustrative of the electron withdrawing groups useful in the present invention include -(C₁-C₆)alkoxy, preferably -(C₁-C₄)alkoxy, further preferably -(C₁-C₂)alkoxy, halogens, -NO₂, -SO₂R₃, -SO₂CH₂F, -SO₂CHF₂, -SO₂CF₃, -S(NSO₂CF₃)CF₃, -CF₃, -CO₂R³, -C(O)CF₃, -CN, cyanovinyl and dicyanovinyl, wherein R³ is a hydrogen or a -(C₁-C₆)alkyl group, preferably -H or a -(C₁-C₄)alkyl group, further preferably -H or a -(C₁-C₂)alkyl group.

The "enantiomeric excess" (ee) is the absolute difference between the mole fractions of the two enantiomers. This value is usually expressed in %ee. An enantioselective reaction is a reaction wherein one of two possible enantiomers is formed in an excess, i.e., an enantiomeric excess can be determined.

The term "subject" as used herein refers to a human or an animal, preferably a mammal such as e.g. non-human primates, mice, rats, rabbits, guinea pigs, dogs, cats, cattle, horses, sheep, pigs, goats and the like. Preferably a "subject" in the context of the present invention is a human.

As mentioned above, a first aspect relates to a method for the preparation of a compound according to Formula IIa 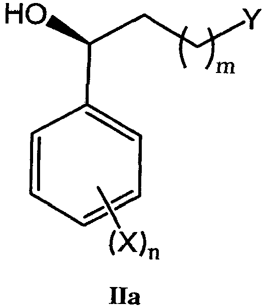 wherein
- n: is 0, 1, 2, or 3;
- X: is, independently of each other, an electron withdrawing group; and
when m = 1:
- Y: is selected from the group consisting of-H (hydrogen), substituted or unsubstituted -(C₁-C₆)alkyl, preferably -(C₁-C₄)alkyl, further preferably -(C₁-C₂)alkyl, -F, -Cl, -Br, -I, -CN, -NH₂, amino substituted by one or two -(C₁-C₆)alkyl groups, preferably -(C₁-C₄)alkyl groups, further preferably -(C₁-C₂)alkyl groups, and a protected amino group; and
when m = 0:
- Y: is -CN;
comprising a step of reducing of a compound according to Formula I 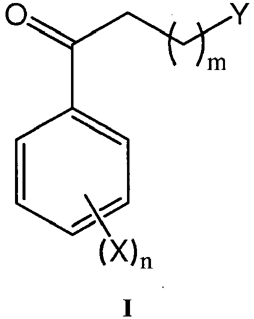 wherein n, X, m and Y are defined as above,
with a xylose reductase selected from the yeast Candida tenuis (AKR 2B5).

In the following, preferred embodiments of a compound of Formula I as defined above are described. It is to be understood that these preferred embodiments of the first aspect of the invention also form preferred embodiments of the compounds of Formula IIa and III, as well as the second aspect of the invention.

In a preferred embodiment, in the above compound according to Formula I, X is, independently, selected from the group consisting of -F, -Cl, -Br, -I, -CN, -NO₂, and (C₁-C₆)alkoxy, and preferably selected from -F, -Cl, -Br, -I, and -CN.

In any of the embodiments wherein n is 2 or 3, each X is independently selected. In other words, when n is 2 or 3, X is present more than once in the compound of Formula I. These different representations of X may be identical or different, and each X may be selected from the corresponding group.

Furthermore, in any of the embodiments wherein n is 1, 2, or 3, X is preferably at the ortho and/or para position in respect to the substituent containing Y. In a further preferred embodiment, X is preferably in the ortho position. In another further preferred embodiment, X is in the para position. The ortho and/or para position is expressed at the phenyl ring containing the substituent X and the group -C(O)CH₂(CH₂)ₘY in respect or in relation to the -C(O)CH₂(CH₂)ₘY group. The ortho position is the position adjacent to the -C(O)CH₂(CH₂)ₘ Y group, and the para position is the opposite or 4-position, if the -C(O)CH₂(CH₂)ₘ Y group is at the 1-position.

In any of the above described embodiments, n is selected from 0, 1, 2, or 3. It is preferred that n is 1 or 2, with 1 being further preferred.

It should be understood that the preferred embodiments referring to X also refer to the preferred embodiments of n and the position of X, and vice-versa.

The compounds according to Formula I are derivatives of 1-phenylpropanone, and comprise 1-phenylpropanone itself. This group of compounds is also referred to as 1-phenylpropanones. To form derivatives of 1-phenylpropanone, when m = 1, Y is selected from the group consisting of hydrogen, substituted or unsubstituted -(C₁-C₆)alkyl, -F, -Cl, -Br, -I, -CN, -NH₂, amino substituted by one or two (C₁-C₆)alkyl groups, and a protected amino group.

In another embodiment, when m = 0, Y is -CN. In this embodiment, the carbon chain contains three carbon atoms, being substituted with an optionally substituted phenyl ring.

In a further preferred embodiment, when m = 1, Y is selected from -H, -Cl, -Br, -NH₂, or methylamino (-NHCH₃).

A central element of the present invention is the reduction of a compound of Formula I to a compound of Formula IIa using a xylose reductase. This reduction is carried out in well known media, like potassium phosphate buffer. In a preferred embodiment, the substituents X and Y are not affected or altered in any way by the reduction using an oxidoreductase.

The reduction of the compound of Formula I is an enantioselective reduction, wherein the enantiomer of the compounds according to Formula IIa is produced at an enantiomeric excess. In a further preferred embodiment, the enantiomeric excess is at least 10 %ee, preferably at least 20 %ee, more preferably at least 50 %ee, more preferably at least 90 %ee, more preferably 95 %ee, even more preferably at least 99 %ee, and most preferably at least 99.5 %ee.

The enantioselective reduction results in the (S)-enantiomer according to Formula Ila 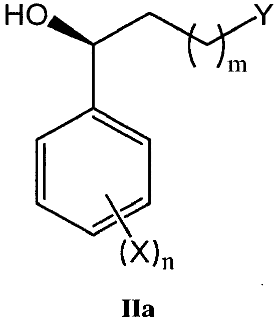 wherein n, X, m and Y are defined as above.

For the methods and uses described herein, the present invention relies on a xylose reductase selected from the yeast *Candida tenuis.*

A xylose reductase from *Candida tenuis* (AKR 2B5) is particularly preferred.

Another particularly preferred embodiment relates to an oxidoreductase selected from the group consisting of:
a) a polypeptide comprising SEQ ID No.: 1;
b) a polypeptide having at least about 70% sequence identity to SEQ ID No.:1 and having xylose reductase activity;
c) fragments or mutants of polypeptides according to a) and b) having xylose reductase activity.

The term "fragment" refers to a polypeptide according to a) and b) as mentioned above which differs from e.g. SEQ ID No.:1 by the deletion of internal amino acids, N-terminal and/or C-terminal truncations.

The person skilled in the art is aware that e.g. a polypeptide of SEQ ID No.: 1 may be shortened at its N- and/or C-termini for about 1, 2, 3, 4, 5, 6, 7, 8, 9, 10 or even more amino acids without substantially affecting its enzymatic activity and specificity. Similarly, there may internal deletions of about 1, 2, 3, 4, 5, 6, 7, 8, 9 or 10 amino acids within SEQ ID No.: 1 without affecting substantially its enzymatic activity and specificity. The term "fragment" thus refers a shortened version of the polypeptides according to a) and b) as mentioned above with the fragments still having a xylose reductase enzymatic activity and specificity comparable to the activity and specificity of those polypeptides from which the fragments are derived. The person skilled in the art will, of course, know which sequences may be particularly suitable for deletion without affecting its enzymatic activity and selectivity. The polypeptide of SEQ ID No.: 1 has been characterized in detail, e.g. in Kratzer et al. (Biochem. J., 393: 51-58, 2006), Pival et al. (FEBS Letters, 582:4095-4099, 2008) and Nidetzky et al. (Biochemistry, 40: 10371-10381; 2001). The person skilled in the art thus has information available which amino acids and amino acid stretches are important for catalytic activity and substrate specificity and which therefore should not be deleted.

The term "mutant" refers to a polypeptide which is based e.g. on SEQ ID No. 1 but which comprises an insertion, e.g. 1, 2, 3, 4, 5, 6, 7, 8, 9, 10 or more additional amino acids without substantially affecting enzymatic activity and specificity. Preferably a polypeptide according to b) is shares at least about 75%, at least about 80%, preferably at least about 85%, at least about 90%, more preferably at least 90%, at least about 91 %, at least about 92%, at least about 93%, at least about 94%, at least about 95% and even more preferably at least about 96%, at least about 97%, at least about 98% and at least about 99% sequence identity with SEQ ID No.: 1.

The determination of percent identity between two protein sequences is preferably accomplished using the mathematical algorithm of Karlin and Altschul (Proc. Natl. Acad. Sci USA 90: 5873-5877, 1993). Such an algorithm is e.g. incorporated into the BLASTp program of Altschul et al. (J. MoI. Biol. 215: 403-410, 1990) available at NCBI (http://www.ncbi.nlm.nih.gov/blast/Blast.cge).

The determination of percent identity is preferably performed with the standard parameters of the BLASTp program.

For the general parameters, the "Max Target Sequences" box may be set to 100, the "Short queries" box may be ticked, the "Expect threshold" box may be set to 10 and the "Word Size" box may be set to "3". For the scoring parameters the "Matrix" box may be set to "BLOSUM62", the "Gap Costs" Box may be set to "Existence: 11 Extension:1", the "Compositional adjustments" box may be set to "Conditional compositional score matrix adjustment". For the Filters and Masking parameters the "Low complexity regions" box may not be ticked, the "Mask for lookup table only" box may not be ticked and the "Mask lower case letters" box may not be ticked. Most prefeably, all parameters are set as outlined above.

It is to be understood that polypeptides which share at least 70% sequence identity with SEQ ID No.:1 must also display xylose reductase activity in order to be a xylose reductase in accordance with the invention.

It will be straightforward for a skilled person to determine whether a polypeptide having e.g. 90% sequence identity with SEQ ID No.: 1 will be a xylose reductase by reacting it with the same substrates as a polypeptide of SEQ ID No.: 1. If the two polypeptides both show enzymatic activity towards these substrates, they will be considered to be xylose reductases.

Examples of polypeptides being derived from SEQ ID No.: 1 include polypeptides which at position 24 of SEQ ID No:1 carry an alanine (W24A), valine (W24V), leucine (W24L), isoleucine (W24I), glycine (W24G), or serine (W24S) instead of tryptophane and/or which at position 51 of SEQ ID No.:1 carry a phenylalanine (D51F) or tyrosine (D51Y) instead of a aspartate. Other mutants which may be introduced in addition or alternatively are W24F and W24Y and/or D51A, D51L, D511 or D51G. The amino acid numbering refers to SEQ ID No.: 1.

As mentioned above, a second aspect of the present invention refers to a method for the preparation of a compound according to Formula III 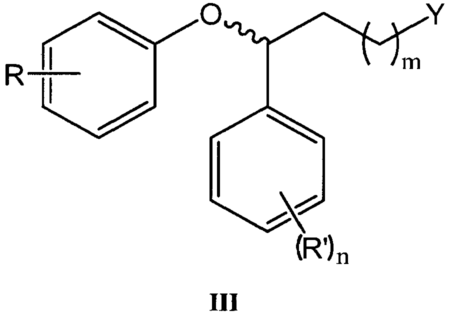 wherein
- n: is 0, 1, 2, or 3;
- R: is selected from the group consisting of substituted or unsubstituted (C₁-C₆)alkyl, substituted or unsubstituted (C₁-C₆)alkoxy; and
- R': is selected from the group consisting of X; and O-SO₃H, -OH, -O-C(O)CH₃, -NH₂, amino substituted by one or two -(C₁-C₆)alkyl groups, -NH-C(O)CH₃, -(C₁-C₆)alkyl, preferabyl -(C₁-C₄)alkyl, further preferably -(C₁-C₂)alkyl, -CH₂OH, -COOH, -COO-(C₁-C₄)alkyl, -CHO, -C(O)-(C₁-C₄)alkyl; and
when m = 1:
- Y: is selected from the group consisting of hydrogen, substituted or unsubstituted -(C₁-C₆)alkyl, preferably -(C₁-C₄)alkyl, further preferably -(C₁-C₂)alkyl, -F, -Cl, -Br, -I, -CN, -NH₂, amino substituted by one or two (C₁-C₆)alkyl groups, and a protected amino group; and
when m = 0:
- Y: is -CN;
comprising the method according to claim 1 for the preparation of a compound according to Formula IIa, and the subsequent transformation of the compound according to Formula IIa to a compound according to Formula III.

The preferred embodiments regarding n, m, X, Y, and the substitution of R' also apply to the compounds of Formula III.

In another preferred embodiment, the transformation comprises the etherification of the compound according to Formula IIa while substantially retaining or inverting the conformation at the asymmetric carbon atom. Thus, a chiral ether is formed. In this context, substantially retaining or inverting the conformation comprises a retention or inversion, respectively, of at least 50 mol-%, preferably at least 70 mol-%, more preferably at least 90 mol-%, even more preferably at least 95 mol-%, and most preferably at least 99 mol-%.

The formation of chiral ethers of any of the intermediate alcohols can be performed in two different ways leading to retention and inversion of the chiral centre in the resulting ethers, respectively.

In a preferred embodiment, a Mitsunobu-type reaction with a substituted phenol using diethylazodicarboxylat (DEAD) causes inversion of the configuration at excellent enantiomeric excesses.

In another preferred embodiment, an ether coupling with a substituted halophenol via NaH used as base leads to retention of the configuration in very good enantiomeric excesses.

In another preferred embodiment, the compounds according to Formula III comprise derivatives of *N*-Methyl-3-aryl-3-aryloxy-propylamines, preferably (*S*)-Fluoxetione, (*S*)-Tomoxetine, (*S*)-Nisoxetine, (*R*)-Fluoxetione, (*R*)-Tomoxetine, and (*R*)-Nisoxetine, as well as the phenyl substituted analogues.

In another preferred embodiment, the compounds according to Formula III comprise derivatives of 3-aryl-3-aryloxy-propylamines, preferably (*S*)-Norfluoxetine, (*R*)-Norfluoxetine, and the phenyl substituted analogues.

The invention is described hereinafter with respect to specific examples. These examples are; however, not to be construed as limiting the invention in any way.

### Example 1: Candida tenuis xylose reductase (CtXR) production

Recombinant xylose reductase of *Candida tenuis* CBS4435 (SEQ ID No.:1) was overexpressed in *Escherichia coli* BL21 (DE3) using the plasmid vector pBeact.li (pet1 la_XR, restriction sites used *Nde*I and *Bam*HI; ampicillin resistance). The bacterial strain was grown in baffled flasks at 37°C and 130 rpm to an OD of 1.0 (600 nm). Enzyme production was induced at 25°C by addition of 250 µM isopropyl-β-D-thiogalactopyranosid. About 18 h after induction, cells were harvested by centrifugation (6000 rpm) and disrupted by 3 passages through a french press. Cell debris was separated by ultra-centrifugation (30,000 rpm) and the cell free raw-extract was subjected to affinity chromatography. Dyes like Red31 resemble the reductase cofactors NAD(P)H and often allow enzyme purification to high purities. Red31 was immobilized on Sepharose CL-4B and used for CtXR purification (J. Chromat. B, 737, 195-202 (2000)). The centrifuged cell extract with a conductivity of 2.5 mS/cm was applied to the Red31 dye-ligand column (6×2.6 cm, approximately 6-8 mg protein/mL of gel) equilibrated with five column volumes of 50 mM potassium phosphate buffer, pH 7.0. Adsorbed protein was eluted at a flow rate of 2 mL/min with a step gradient of 0.2 M and 2 M NaCl. Fractions of 20 mL were collected and the 2 M NaCl peak containing most of the *Ct*XR, as judged by activity measurements and SDS-PAGE, was pooled. The pooled fractions were concentrated in Vivaspin 20 mL concentrator tubes with 10,000 molecular weight cut off to a volume of less than 10 mL. The buffer was changed to 50 mM potassium phosphate buffer, pH 7.0 without NaCl and re-concentrated. Purification grade was monitored by sodium dodecyl sulphate-polyacrylamide gel electrophoresis.

### Example 2: Kinetic parameter determination

Initial rate measurements were carried out as described recently *(*Biochem. J., 385, 75-83 (2005)). Keto substrates were dissolved in ethanol or DMSO prior to dilution into 50 mM potassium phosphate buffer, pH 7.0 to a final ethanol concentration of 5 % ethanol or 25 % DMSO. Kinetic parameters for enzymatic ketone reduction by NADH were not affected by the added ethanol or DMSO. They were obtained under conditions in which the substrate concentration was varied and the coenzyme concentration was constant and saturating (carbonyl reduction, 280 µM NADH). The enzyme concentration in the assays was in the range of 0.03-10.0 µM, depending on the activity towards the respective substrate. Appropriate controls containing enzyme and coenzyme, or the substrate and coenzyme were determined under conditions otherwise exactly identical to the enzymic assay. If required, the initial rates were corrected for blank readings. All experiments were performed at 25°C. Data processing and statistical analysis were carried out as reported elsewhere *(*Biochem. J., 385, 75-83 (2005)). The limited solubility of certain compounds often prevented saturation of the enzymes with ketone substrates. In these cases, the catalytic efficiency was obtained from the part of the Michaelis-Menten plot where under conditions of [substrate] « Km, the reaction rate is linearly dependent on [substrate] with a slope that equals (*k*_{cat}/*K*ₘ) divided by the molar concentration of enzyme.

### Example 3: CtXR catalyzed transformations of 1-phenylpropanones to the corresponding (S)-1-phenylpropanoles

The *Ct*XR, *CtXR* W24F, and *CtXR* D51A catalyzed reductions are performed using 9.5 units of the respective biocatalyst in potassium phosphate buffer (300 mL; 50 mM; pH 7.5; 10% ethanol; 25°C). To each transformation the prochiral substrates 3-aminopropiophenone, 3-chloropropiophenone, 3-bromopropiophenone, 3-iodopropiophenone, or 3-phenyl-3-oxo-propannitrile are added in 10 mM concentration and additionally NADH is added in 12 mM concentration. Alternatively the transformations are performed in potassium phosphate buffer (300 mL; 50 mM; pH 7.5; 10% ethanol; 25°C) with substrate (10 mM), ammonium formiate (40 mM), NAD(H) (230 µM), CtXR, *Ct*XR W24F, or *CtXR* D51A (10 units), and formate dehydrogenase (45 units) for recycling of the NADH. The course of transformations are monitored by UV-spectroscopy (290 nm) and stopped by addition of sodium azide (0.3 mM) when the conversion finished. The enzymes are separated by a 76 mm ultrafiltration stirred cell with a cut-off of 10,000 Da. The aqueous layer is extracted three times with CH₂Cl₂ (150 mL each) and combined organic extracts are washed with brine and dried over MgSO₄. The solutions are evaporated to dryness to get the crude reaction mixture for determination of the conversions. Subsequent silica gel column chromatography (CH₂Cl₂/ethylacetate 10:1) afforded the extracted (*S*)-3-aryl-3-hydroxylpropylamines, (*S*)-1-chloro-3-aryl-3-hydroxylpropane, (*S*)-1-bromo-3-aryl-3-hydroxylpropane, (*S*)-1-iodo-3-aryl-3-hydroxylpropane and (*S*)-3-phenyl-3-hydroxy-propannitrile as yellow oil or amorphous solid, respectively.

### Example 4: Analysis of reduction products

Keto function containing substrates were dissolved in ethanol or DMSO prior to dilution into 50 mM potassium phosphate buffer, pH 7.0 to a final ethanol concentration of 5 % ethanol or 25 % DMSO. 1 mM excess [NADH] and about 0.4-2.4 units of enzyme activity, measured with the substrate to be reduced were added and the reaction mixture was incubated at 25°C. After keto reduction of ~ 80 % (< 5-25 min), determined as the depletion of NADH, the solutions were centrifuged prior to chromatographic analysis.

The structures of the resulting products were determined by NMR spectroscopy. Therefore saturated aqueous NaHCO₃ solution was added and the mixture was extracted three times with ethyl acetate. Combined organic extracts were washed with H₂O and saturated aqueous NaCl solution, dried over Na₂SO₄, and concentrated *in vacuo.* ¹H and ¹³C NMR spectra were measured at 298.15 K in CDCl₃ (>99.98% D) on a Bruker Avance DRX-400 or DRX-600 at 400.13 MHz (100.61 MHz) or 600.13 MHz (150.90 MHz), respectively. Chemical shifts were referenced to residual CHCl₃ (δ_{H} = 7.26) and CDCl₃ (δ_{C} = 77.36) and are given in ppm. Assignments of proton resonances were confirmed by two dimensional homonuclear (COSY, TOCSY, NOESY) and heteronuclear (HSQC, HMBC) spectroscopy.

### Example 5: Site directed mutagenesis

Site-directed mutagenesis of *CtXR* was carried out by using inverse PCR that followed a published protocol with slight modifications *(*Nucleic Acids Res., 17, 6545-6551 (1989)). The plasmid vector pBEAct.li *(*Biol. Chem., 380, 1395-1403 (1999)) was directly amplified with Pfu DNA polymerase and the two oligonucleotide primers listed below with the mismatched bases underlined. The primers were phosphorylated at the 5'end using T4 polynucleotide kinase prior to PCR amplification.
D51A forward: 5' GAGGCCTACGGTAACGAAAAG 3' (SEQ IDNo.:2)
D51 reverse: 5' GGCACCGTCGAACAATCTGTA 3' (SEQ ID No.:3)
W24F forward: 5' GGTTTCGGCTGTTTCAAACTCG 3' (SEQ ID No.:4)
W24 reverse: 5' GATGGATGGCATTAAGTGGCCG 3' (SEQ ID No.:5)

To facilitate colony screening by restriction site analysis, a Fokl restriction site in the W24 reverse primer was inserted by introducing silent mutations. Introduction of additional silent mutations avoided dimerisation of oligonucleotides. The amplified linear plasmid was gel purified and ligated using a T4 DNA ligase. The ligation product was desalted over a MF-membranefilter (pore size 0.025 µm, diameter 13 mm, Millipore) for 1 hour prior to electroporation into competent *E. coli* BL21 (DE3) cells. Plasmid miniprep DNA was subjected to dideoxy sequencing to verify the introduction of the desired mutations and that no misincorporations of nucleotides had occurred because of DNA polymerase errors. Purified D51A, W24F mutants migrated in SDS/PAGE as single protein bands to exactly the same position as the wild-type.

### Example 6: Friedel Crafts acylation of benzene and substituted analogues

A mixture of 4-chlorobenzene (3.9 g, 34 mmol) and AlC13 (2 g, 15 mmol) is cooled to 0-5°C and stirred for 30 min. before 3-chloropropionyl chloride (1 g, 13 mmol) is added dropwise. After 30 min the resulting solution is heated to 50°C for 12 h. The reaction mixture is poured into 300 mL of a icewater/HCl-mixture and extracted with CH₂Cl₂ (3 x 50 mL). The combined organic extracts are washed with 10% HCl-, NaCl-, NaHCO₃- and sat. NaCl-solutions and dried over MgSO₄. The solvent is removed via distillation (<50°C) and the resulting is purified by chromatography (hexan/ethylacetate 8:2) to gain 3-chloro-1-(4-chlorophenyl)-1-propanone (4.06 g, 20 mmol, 60 %: Organikum, 20. Aufl.: Johann Ambrosius Barth Verlag, Heidelberg, p. 362 (1996)).

### Example 7: 3-Hydroxypropiophenones via side chain elongation of acetophenones

A mixture of 2,4-dichloroacetophenone (1 g, 5 mmol) and 37% formaldehyde (0.3 g, 10 mmol) are placed in sealed vessel in a microwave cavity. Microwave irradiation of 250 W is used, which elevates the temperature from 25°C to 175°C during 5 min. The reaction mixture is held at this temperature for 8 min. After cooling, reaction mixture is extracted with chloroform. The organic extracts are washed with brine, dried, and filtered. Evaporation of the solvent provides a residue that is purified by chromatography on silica gel with CH₂Cl₂ to furnish 1-(2,4-Dichlorophenyl)-3-hydroxypropanone (0.38 g, 1,8 mmol, 35 %; J. Med. Chem., 51, 3841-3855 (2008)).

### Example 8: Side chain elongation of acetophenone and substituted analogues

A mixture of 4-bromoacetophenone (0.1 g, 0.5 mmol), methylamine hydrochloride (0.1 g, 1 mmol), paraformaldehyde (0.05 g, 1.7 mmol), and conc. HCl (0.1 g) in 1 mL ethanol was heated in a sealed flask at 110°C for 24 h. After cooling to RT, the solvent was removed and 5 mL of ethyl acetate was added. The resulting suspension was vigorously stirred at RT for 6 h and then filtered and washed with ethyl acetate to afford 1-(4-Bromophenyl)-3-methylaminopropan-1-one hydrochlorid (0.07 g, 0.25 mmol, 50 %; Angew. Chem. Int. Ed., 44, 1687-1689 (2005)).

### Example 9: Synthesis of 3-phenyl-3-oxo-propionitriles from benzoic acids

2-Chlorobenzoic acid (0.3 g, 1.9 mmol) and Potassium carbonate (1.3 g, 9.3 mmol) are solved in acetone. Methyliodide (2.3g, 15 mmol) is dropwise added to this solution. This mixture is heated with a reflux condenser for 18 h. After filtration with a silica plug the solvent is distilled under reduced pressure to gain methyl 2-chlorobenzoate (0.3 g, 1.9 mmol, 91 %).

To a lithium diisopropylamide solution (2.0 g, 3 mmol, 1.5 M) in 6 mL THF acetonitrile (0.075 g, 1.8 mmol) is added dropwise at -78°C. After the solution has been stirred at -78°C for 1 h, methyl 2-chlorobenzoate (0.028 g, 1.7 mmol) in 6 mL THF is added dropwise at -78°C. The reaction mixture is kept at -78°C for 1 h and warmed up to RT. After being stirred for an additional 2 h, the reaction is quenched by addition of H₂O. The aqueous layer is acidified to pH 5 with 1 N HCl and extracted three times with ether. The combined organic extracts are dried over Na₂SO₄ and concentrated to give 3-(2'-chloro)-phenyl-3-oxo-propionitrile (0.025 g, 1.5 mmol, 85 %; Bioorg. Med. Chem. 2006, 14, 7501-7511.).

### Example 10: 3-N-Methylaminopropiophenones from phenyl-3-oxo-propannitriles

A solution of NaBH₄ (0.06 g, 1.5 mmol) in dry THF(10 mL) is added dropwise to a solution of ketal protected phenyl-3-oxo-propionitrile (0.3 g, 1.5 mmol) in dry THF (10 mL) at 0°C with stirring under Ar. The mixture is heated at 70°C for 6 h. After cooling to 0°C 20 mL water is added to the reaction which is then extracted with ethylacetate (3 x 15 mL). The combined organic extracts are dried over MgSO₄ and concentrated *in vacuo.* Purification by chromatography (ethylacetate/methanol 1:1) provides ketal protected aminopropiophenone as a colorless solid (0.27 g, 1.3 mmol, 85 %), which is then deprotected under acid conditions.

To a solution of aminopropiophenone (0.2 g, 1.3 mmol) and methyl chloroformate (1.4 g, 1.5 mmol) in CH₂Cl₂ (15 mL) is K₂CO₃ (7.2 g, 5.2 mmol) in H₂O (15 mL) added with stirring at 0°C. The mixture is warmed to RT, stirred further for 1 h. 10 mL water are added and the mixture is extracted with CH₂Cl₂ (2x10 mL). Combined organic extracts are dried over MgSO₄ and concentrated *in vacuo*. The residual is dissolved in 10 mL THF and the resulting solution is directly used in the reduction. Therefore to a suspension of LiAlH₄ (0.5 g, 1.3 mmol) in dry THF (20 mL) is a solution of the above formamide intermediate in 10 mL of THF added dropwise at 0°C with stirring under Ar. Then the mixture is heated at reflux for 12 h. After cooling to 0°C, 4 mL of degassed water is added to quench the reaction. The resulting mixture is filtered, and the organic layers are separated, dried over MgSO₄ and concentrated *in vacuo.* Purification by chromatography on silica gel chromatography (CH₂Cl₂/methanol 1:1) yielded 3-N-methylaminopropiophenone as a colorless oil (0.16 g, 1.0 mmol, 80 %; Org. Biomol. Chem., 3, 2513-2518 (2005)).

### Example 11: The formation of chiral ethers

a.) Diethylazodicarboxylat (DEAD, 0.03 g, 0.17 mmol) and triphenylphosphine (0.05 g, 0.19 mmol) are added to a solution of (*S*)-1-chloro-3-hydroxyl-3-phenylpropanes (0.28 g, 0.16 mmol) and *ortho*-cresol (0.02 g, 0.18 mmol) in 1 mL THF. The mixture is stirred at RT for 16 h. THF is removed under vacuum and the residue extracted with hexane (3 x 50 mL). The combined hexane fractions are concentrated, and the residue is purified on silica gel chromatography (hexane/EtO₂ 1: 1) provide (R)-1-chloro-3-(2'-methylphenyloxy)-3-phenylpropane as a colourles liquid (0.029 g, 0.11 mmol, 70 %; J. Chem. Soc, Perkin Trans., 1, 1767-1769. (2000)).
b.) 60% NaH (1.19 g, 30 mmol) is added in four batches into a solution of (S)-1-chloro-3-hydroxyl-3-phenylpropanes (4.1 g, 25 mmol) in DMSO (20 mL) at 0°C with stirring. After the mixture has been heated to 70°C for 30 min, a solution of 4-chlorobenzotrifluoride (4.5 g, 25 mmol) in DMSO (5 mL) is added to the mixture and then heated at 90°C for 2 h. The resulting mixture is cooled to 0°C, water (20 mL) is added and extracted with ether (2 x 20 mL). The ether extracts are combined and dried over MgSO₄ and concentrated to about 10 mL. A saturated solution of HCl in ether was added dropwise into the solution to provide (S)-1-chloro-3-(2'-methylphenyloxy)-3-phenylpropane as colorless oil (7.79 g, 22.5 mmol, 90 %; Org. Biomol. Chem., 3, 2513-2518 (2005)).

### Example 12: Reduction of 3-aryloxy-3-phenyl-propionitriles

A solution of NaBH₄ (0.026 g, 0.7 mmol) in dry THF(10 mL) is added dropwise to a solution of (S)-3-(2'-methylphenyloxy)-3-phenylpropannitrile (0.1 g, 0.7 mmol) in dry THF (10 mL) at 0°C with stirring under Ar. The mixture is heated at 70°C for 6 h. After cooling to 0°C 20 mL water is added to the reaction which is then extracted with ethylacetate (3 x 15 mL). The combined organic extracts are dried over MgSO₄ and concentrated *in vacuo.* Purification by chromatography (ethylacetate/methanol 1:1) provide (S)-3-(2'-methylphenyloxy)-3-phenylpropylamine as a white solid (0.09 g, 0.63 mmol, 90 %, Org. Biomol. Chem., 3, 2513-2518 (2005)).

### Example 13: N-Methyl-3-aryloxy-3-phenylpropylamines from 3-aryloxy-3-phenylpropylamines

To a solution of (S)-3-(2'-methylphenyloxy)-3-phenylpropylamine (0.05 g, 0.3 mmol) and methyl chloroformate (0.04 g, 0.4 mmol) in CH₂Cl₂ (15 mL) is K₂CO₃ (0.07 g, 1.3 mmol) in H₂O (15 mL) added with stirring at 0°C. The mixture is warmed to RT, stirred further for 1 h. 10 mL water are added and the mixture is extracted with CH₂Cl₂ (2 x 10 mL). Combined organic extracts are dried over MgSO₄ and concentrated *in vacuo.* The residual is dissolved in 10 mL THF and the resulting solution is directly used in the reduction. Therefore to a suspension of LiAlH₄ (0.01 g, 0.3 mmol) in dry THF (20 mL) is a solution of the above formamide intermediate in 10 mL of THF added dropwise at 0°C with stirring under Ar. Then the mixture is heated at reflux for 12 h. After cooling to 0°C, 4 mL of degassed water is added to quench the reaction. The resulting mixture is filtered, and the organic layers are separated, dried over MgSO₄ and concentrated *in vacuo.* Purification by chromatography on silica gel chromatography (CH₂Cl₂/methanol 1:1) yielded (*S*)-*N*-methyl-3-(2'-methylphenyloxy)-3-phenylpropylamine as a white solid (0.05 g, 0.29 mmol, 90 %, Org. Biomol. Chem., 3, 2513-2518 (2005)).

### Example 14: N-methyl-3-aryloxy-3-phenylpropylamines from 1-halo-3-aryloxy-3-phenylpropanes

(*R*)-1-Chloro-3-(2'-methylphenyloxy)-3-phenylpropane (0.028 g, 0.09 mmol) and aqueous MeNH₂ (40%, 0.5 mL) are dissolved in 5 mL ethanol. The solution is refluxed in a close vessel at 120°C for 12 h, cooled to RT and poured into water. The mixture is extracted with ether (3 x 20 mL) and the organic extract is washed with water and aq. NaCl, dried over MgSO₄ and concentrated *in vacuo.* The residue is purified by chromatography (CH₂Cl₂/MeOH 10:1) to yield (*S*)-*N*-methyl-3-(2'-methylphenyloxy)-3-phenylpropylamine *hydrochlorid* (0,015 g, 0,05 mmol, 60 %; J. Chem. Soc, Perkin Trans. 1, 1767-1769 (2000)).

## Claims

1. Method for the preparation of a compound according to Formula IIa 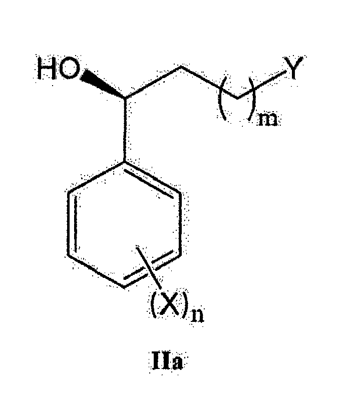 wherein
n is 0, 1, 2, or 3;
m is 0 or 1;
X is, independently of each other, an electron withdrawing group; and
when m = 1:
Y is selected from the group consisting of -H, substitsted or unsubstiuted -(C₁-C₆)alkyl -F, -Cl, -Br, -I, -CN, -NH₂, amino substituted by one or two (C₁-C₆)alkyl groups, and a protected amino group; and
when m = 0:
Y is -CN;
comprising a step of reducing a compound according to Formula I 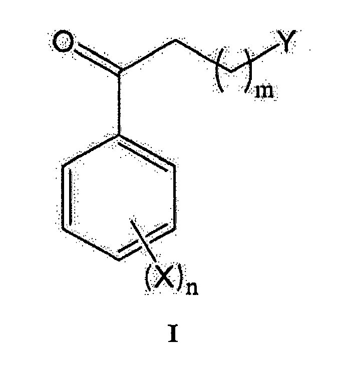 wherein n, X, m and Y are defined as above,
with a xylose reductase selected from the yeast *Candida tenuis* (AKR 2B5).

2. Method according to claim 1, wherein X is, independently, selected from the group consisting of -F, -Cl, -Br, -I, and -CN.

3. Method according to any of the previous claims, wherein n is 1 or 2.

4. Method according to any of the previous claims, wherein Y is -H, -Cl, -Br, -NH₂, or -NHCH₃.

5. Method for the preparation of a compound according to Formula III 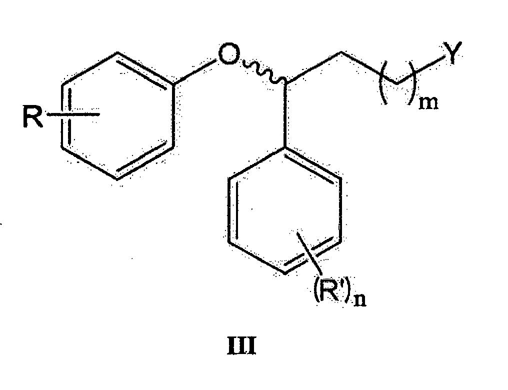 wherein
n is 0, 1, 2, or 3;
m is 0 or 1;
X is, independently of each other, an electron withdrawing group;
R is selected from the group consisting of substituted or unsubstituted (C₁-C₆)alkyl, substituted or unsubstituted (C₁-C₆)alkoxy; and
R' is selected from the group consisting of X; and O-SO₃H, -OH, -O-C(O)CH₃, -NH₂, amino substituted by one or two -(C₁-C₆)alkyl groups, -NH-C(O)CH₃, -(C₁-C₆)alkyl, -CH₂OH, -COOH, -COO-(C₁-C₄)alkyl, -CHO, -C(O)-(C₁-C₄)alkyl; and
when m = 1:
Y is selected from the group consisting of -H, substituted or unsubstituted -(C₁-C₆)alkyl, -F, -Cl, -Br, -I, -CN; -NH₂, amino substituted by one or two (C₁-C₆)alkyl groups, and a protected amino group; and
when m = 0:
Y is -CN;
comprising the method according to any of the previous claims for the preparation of a compound according to Formula IIa, and the subsequent transformation of the compound according to Formula IIa to a compound according to Formula III.

6. Method according to claim 5, wherein the transformation comprises the etherification of the compound according to Formula IIa while substantially retaining or inverting the conformation at the asymmetric carbon atom.

7. Method according to any of the previous claims, wherein said xylose reductase selected from the yeast *Candida tenuis* (AKR 2B5) is selected from the group consisting of:
a) a polypeptide comprising SEQ ID No.: 1;
b) a polypeptide having at least 70% sequence identity to SEQ ID No.:1 and having xylose reductase activity;
c) fragments or mutants of polypeptides according to a) and b), wherein a fragment is a polypeptide which differs from SEQ ID No.: 1 by the deletion of internal amino acids, N-terminal and/or C-terminal truncations without affecting the enzymatic activity and specificity of SEQ ID No.: 1 and wherein a mutant is a polypeptide which differs from SEQ ID No.: 1 by insertion of one or more additional amino acids without affecting the enzymatic activity and specificity of SEQ ID No.: 1.

8. Use of a xylose reductase selected from the yeast *Candida tenuis* for the reduction of a compound according to Formula I

9. Use according to any of claims 8, wherein said xylose reductase selected from the yeast *Candida tenuis* is selected from the group consisting of:
a) a polypeptide comprising SEQ ID No.: 1;
b) a polypeptide having at least 70% sequence identity to SEQ ID No.:1 and having xylose reductase activity;
c) fragments or mutants of polypeptides according to a) and b), wherein a fragment is a polypeptide which differs from SEQ ID No.: 1 by the deletion of internal amino acids, N-terminal and/or C-terminal truncations without affecting the enzymatic activity and specificity of SEQ ID No.: 1 and wherein a mutant is a polypeptide which differs from SEQ ID No.: 1 by insertion of one or more additional amino acids without affecting thee enzymatic activity and specificity of SEQ ID No.: 1.

## Patentansprüche

1. Verfahren zur Herstellung einer Verbindung gemäß Formel IIa 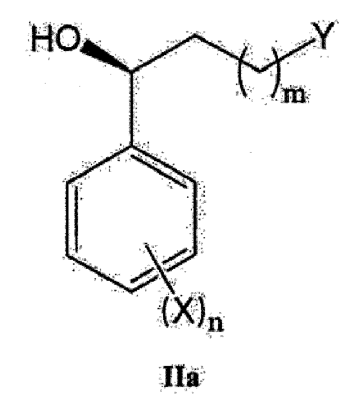 wobei
n 0, 1, 2, oder 3 ist,
m 0 oder 1 ist
X unabhängig voneinander einer elektronenentziehende Gruppe entspricht; und
wenn m = 1:
Y aus der Gruppe ausgewählt ist, bestehend aus -H, substituiert oder nicht substituiert mit -(C₁-C₆)alkyl, -F, -Cl, -Br, -I, -CN, -NaH2, amino substituiert durch eine oder zwei (C₁-C₆)alkyl-Gruppen und einer geschützten Aminogruppe; und
wenn m = 0:
Y -CN entspricht;
umfassend einen Schritt die Verbindung gemäß Formel I 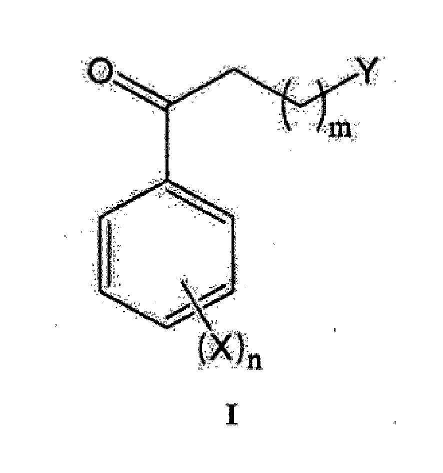 wobei n, X, m und Y wie oben definiert sind,
mit einer Xylose Reduktase ausgewählt aus der Hefe *Candida tenuis* (AKR 2B5) zu reduzieren

2. Verfahren gemäß Anspruch 1, wobei X, unabhängig, aus der Gruppe ausgewählt ist, bestehend aus -F, -Cl, -Br, -I, and -CN.

3. Verfahren gemäß einem der vorhergehenden Ansprüche, wobei n 1 oder 2 entspricht.

4. Verfahren gemäß einem der vorhergehenden Ansprüche, wobei Y, -H, -Cl, -Br, -NH₂, or -NHCH₃ entspricht.

5. Verfahren zur Herstellung einer Verbindung gemäß Formel III 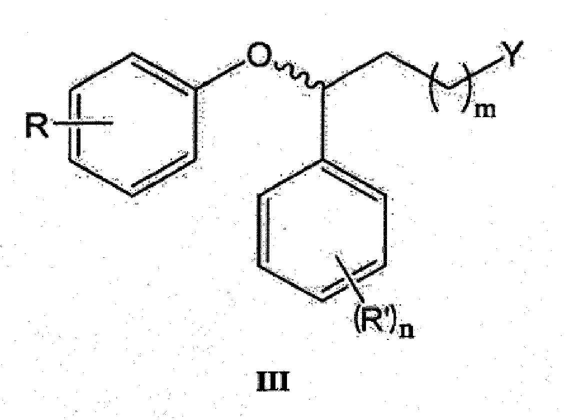 wobei
n 0, 1, 2, oder 3 ist,
m 0 oder 1 ist
X unabhängig voneinander einer elektronenentziehende Gruppe entspricht;
R aus der Gruppe ausgewählt ist, bestehend aus substituierten oder nicht substituierten (C₁-C₆)alkyl, substituierten oder nicht substituierten (C₁-C₆)alkoxy; und
R¹ aus der Gruppe ausgewählt ist, bestehend aus X; und O-SO₃H, -OH, -O-C(O)CH₃, -NH₂, amino substituiert durch eine oder zwei -(C₁-C₆)alkyl Gruppen, -NH-C(O)CH₃, (C₁-C₆)alkyl, -CH₂OH, -COOH, -COO-(Ci-C4)alkyl, -CHO, -C(O)-(C₁-C₄)alkyl; und
wenn m = 1
Y aus der Gruppe ausgewählt ist, bestehend aus -H, substituiert oder nicht substituiert -(C₁-C₆)alkyl, -F, -Cl, -Br, -I, -CN, -NH₂, amino substituiert durch eine oder zwei (C₁-C₆)alkyl Gruppen und einer geschützten Aminogruppe; und
wenn m = 0
Y -CN entspricht;
umfassend das Verfahren gemäß einem der vorhergehenden Ansprüche für die Herstellung einer Verbindung gemäß Formel IIa, und der darauffolgenden Umwandlung der Verbindung gemäß Formel IIa in eine Verbindung gemäß Formel III.

6. Verfahren gemäß Anspruch 5, wobei die Umwandlung die Veretherung der Verbindung gemäß Formel IIa umfasst, während die Asymmetrie am Kohlenstoffatom im Wesentlichen erhalten bleibt oder umgekehrt wird.

7. Verfahren gemäß einem der vorhergehenden Ansprüche, wobei die Xylose Reduktase ausgesucht aus der Hefe *Candida tenuis* (AKR 2B5) ausgewählt ist aus der Gruppe bestehend aus:
a) einem Polypeptid umfassend die SEQ ID No.: 1
b) einem Polypeptid, welches mindestens 70% Sequenzidentität zur SEQ ID No.: 1 aufweist und Xylose Reduktase Aktivität besitzt;
c) Fragmente oder Mutanten von Polypeptiden gemäß a) und b), wobei ein Fragment ein Polypeptid ist, welches sich von SEQ ID No.: 1 durch das Fehlen einer internen Aminosäure, durch N-terminale und/oder C-terminale Verkürzungen unterscheidet, ohne die enzymatische Aktivität oder Spezifität der SEQ ID No.: 1 zu beeinflussen und wobei eine Mutante ein Polypeptid ist, welches sich von SEQ ID No.: 1 durch Einfügen einer oder mehrerer, zusätzlicher Aminosäuren unterscheidet, ohne die enzymatische Aktivität oder Spezifität der SEQ ID No.: 1 zu beeinflussen.

8. Verwendung einer Xylose Reduktase ausgewählt aus der Hefe *Candida tenuis* zur Reduktion der Verbindung gemäß Formel I,

9. Verwendung gemäß Anspruch 8, wobei die Xylose Reduktase ausgesucht aus der Hefe *Candida tenuis* ausgewählt ist aus der Gruppe bestehend aus:
a) einem Polypeptid umfassend die SEQ ID No.: 1
b) einem Polypeptid, welches mindestens 70% Sequenzidentität zur SEQ ID No.: 1 aufweist und Xylose Reduktase Aktivität besitzt;
c) Fragmente oder Mutanten von Polypeptiden gemäß a) und b), wobei ein Fragment ein Polypeptid ist, welches sich von SEQ ID No.: 1 durch das Fehlen einer internen Aminosäure, durch N-terminale und/oder C-terminale Verkürzungen unterscheidet, ohne die enzymatische Aktivität oder Spezifität der SEQ ID No.: 1 zu beeinflussen und wobei eine Mutante ein Polypeptid ist, welches sich von SEQ ID No.: 1 durch Einfügen einer oder mehrerer, zusätzlicher Aminosäuren unterscheidet, ohne die enzymatische Aktivität oder Spezifität der SEQ ID No.: 1 zu beeinflussen.

## Revendications

1. Procédé de préparation d'un composé selon la formule IIa 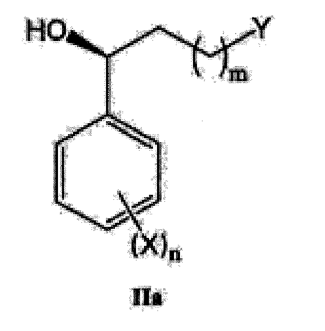 dans laquelle
n vaut 0, 1, 2, ou 3 ;
m vaut 0 ou 1;
X est, indépendamment l'un de l'autre, un groupe attracteur d'électrons ; et
lorsque m = 1 :
Y est choisi dans le groupe constitué par -H, un alkyle(C₁-C₆) substitué ou non substitué, -F, -CI, -Br, -I, -CN, -NH₂, un amino substitué par un ou deux groupes alkyle(C₁-C₆) et un groupe amino protégé ; et
lorsque m = 0 :
Y est -CN ;
comprenant une étape de réduction d'un composé selon la formule I 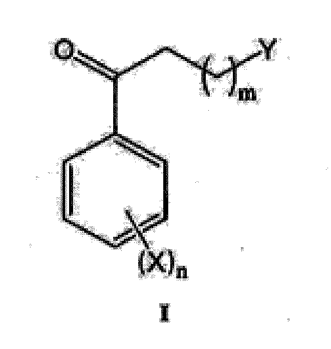 dans laquelle n, X, m et Y sont définis comme ci-dessus,
avec une xylose réductase choisie chez la levure *Candida tenuis* (AKR 2B5).

2. Procédé selon la revendication 1, dans lequel X est, indépendamment, choisi dans le groupe constitué par -F, -CI, -Br, -I et -CN.

3. Procédé selon l'une quelconque des revendications précédentes, dans lequel n vaut 1 ou 2.

4. Procédé selon l'une quelconque des revendications précédentes, dans lequel Y est -H, -CI, -Br, -NH₂ ou NHCH₃.

5. Procédé de préparation d'un composé selon la formule III 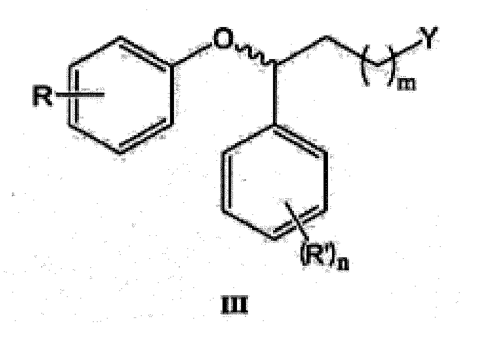 dans laquelle
n vaut 0, 1, 2 ou 3
n vaut 0 ou 1 ;
X est, indépendamment l'un de l'autre, un groupe attracteur d'électrons ;
R est choisi dans le groupe constitué par un alkyle(C₁-C₆) substitué ou non substitué, un alcoxy(C₁-C₆) substitué ou non substitué ; et
R' est choisi dans le groupe constitué par X; et O-SO₃H, -OH, -OC(O)CH₃, -NH₂, un amino substitué par un ou deux groupes alkyle(C₁-C₆), -NH-C(O)CH₃, un alkyle(C₁-C₆), -CH₂OH, -COOH, -COO-alkyle(C₁-C₄), -CHO, -C(O)-alkyle(C₁-C₄) ;
et
lorsque m = 1 :
Y est choisi dans le groupe constitué par -H, un alkyle(C₁-C₆) substitué ou non substitué, -F, -CI, -Br, -I, -CN, -NH₂, un amino substitué par un ou deux groupes alkyle(C₁-C₆) et un groupe amino protégé ; et
lorsque m = 0 :
Y est -CN ;
comprenant le procédé selon l'une quelconque des revendications précédentes pour la préparation d'un composé de formule IIa, et la transformation subséquente du composé selon la formule IIa en un composé selon la formule III.

6. Procédé selon la revendication 5, dans lequel la transformation comprend l'éthérification du composé selon la formule IIa tout en retenant essentiellement ou en inversant la conformation à l'atome de carbone asymétrique.

7. Procédé selon l'une quelconque des revendications précédentes, dans lequel ladite xylose réductase choisie chez la levure *Candida tenuis* (AKR 2B5) est choisi dans le groupe constitué par :
a) un polypeptide comprenant SEQ ID n° 1 ;
b) un polypeptide ayant au moins 70 % d'identité de séquence à SEQ ID n° 1 et ayant une activité xylose réductase ;
c) des fragments ou des mutants de polypeptides selon a) et b), dans lesquels un fragment est un polypeptide qui diffère de SEQ ID n° 1 par la délétion d'acides aminés internes, des troncatures N-terminales et/ou C-terminales sans affecter l'activité enzymatique et la spécificité de SEQ ID n° 1 et dans lesquels un mutant est un polypeptide qui diffère de SEQ ID n° 1 par l'insertion d'un ou de plusieurs acides aminés supplémentaires sans affecter l'activité enzymatique et la spécificité de SEQ ID n°1.

8. Utilisation d'une xylose réductase choisie chez la levure *Candida tenuis* pour la réduction d'un composé selon la formule I.

9. Utilisation selon l'une quelconque des revendications 8, dans laquelle ladite xylose réductase choisie chez la levure *Candida tenuis* est choisie dans le groupe constitué par :
a) un polypeptide comprenant SEQ ID n° 1;
b) un polypeptide ayant au moins 70 % d'identité de séquence à SEQ ID n° 1 et ayant une activité xylose réductase ;
c) des fragments ou des mutants des polypeptides selon a) et b), dans lesquels un fragment est un polypeptide qui diffère de SEQ ID n° 1 par la délétion d'acides aminés internes, de troncatures N-terminales et/ou C-terminales sans affecter l'activité enzymatique et la spécificité de SEQ ID n° 1 et dans lesquels un mutant est un polypeptide qui diffère de SEQ ID n° 1 par l'insertion d'un ou de plusieurs acides aminés supplémentaires sans affecter l'activité enzymatique et la spécificité de SEQ ID n° 1.
